# EUROPEAN PATENT SPECIFICATION

(11) **EP 0 412 789 B1**
(45) Date of publication and mention of the grant of the patent: **03.01.1996**
(21) Application number: 90308709.6
(22) Date of filing: 08.08.1990
(51) Int. Cl.: A61F 9/00

(54) **Laser ablation of surfaces**
Laser-Abschmelzung von Oberflächen
Ablation de surface avec laser

(30) Priority: 11.08.1989 US 393516
(43) Date of publication of application: 13.02.1991
(73) Proprietor: SUMMIT TECHNOLOGY, INC., Watertown, Massachusetts 02172 (US)
(72) Inventor: Muller, David F., Boston, Massachusetts 02199 (US); Klopotek, Peter J., Framingham, Massachusetts 01710 (US)
(74) Representative: Harvey, David Gareth

(56) References cited:
- EP-A- 0 274 205
- US-A- 4 648 400

## Description

This invention relates to laser ablation of surfaces, especially but not exclusively surfaces of biological materials. In particular, the invention relates to systems for reprofiling the cornea of the eye. Moreover, the invention relates to methods of shaping surfaces which are not part of the living body of a human being or animal.

A known surgical technique for treatment of some forms of refractive errors, e.g., acute myopia, hyperopia, and astigmatism, comprises surgically removing the defective anterior segment of the cornea down into the corneal stroma, reshaping the removed segment via surgical grinding in a frozen state, and restoring the reshaped segment onto the eye. In this type of operation, known as keratoplasty, the eye heals by reformation of the outer epithelium layer over the reshaped corneal stroma.
Alternatively, an incision can be made into the Bowman's membrane in the form of a flap, an artificial or donor lenticular implant inserted under the flap, and the flap sutured closed. In this latter operation, as the eye heals the implant becomes engrafted into the structure of the cornea, remediating its curvature.

Other surgical techniques for correcting refractive errors by altering the shape of the corneal surface have also been proposed. For example, in radial keratectomy a set of radial incisions, resembling the spokes of a wheel, are made in the cornea. As the incisions heal, the curvature of the eye is flattened, thereby increasing the ocular focal distance. Although an effective treatment for refractive errors such as myopia (nearsightedness), the operation is not particularly suitable for correction of hyperopia (farsightedness) and can pose problems if the surgical incisions are uneven or too deep.

The above-described surgical operations are often carried out using diamond or steel scalpels. (See, generally, Binder et al, "A Refractive Keratoplasty," 100 Arch. Ophthalmol., 802 (1982) and "Refractive Keratoplasty Improves With Polysulfone Pocket Incision," Opthalmology Times (July 1, 1986)).

The use of the laser beam as a surgical tool for cutting non-ocular incisions, a so-called "laser scalpel", has been known for some time. (See, for example, U.S. Patent 3,769,963 to Goldman et al). The utility of most forms of laser radiation for corneal surgery has been limited, however, by the tendency of laser beams to cause thermal damage and related scaring, as well as opacification, in the extremely delicate structure of the cornea. Accordingly, the "cold" photoablative UV radiation from excimer lasers and the like must be carefully controlled to avoid permanent damage to the eye. (For a study of the damage which can be inflicted on the cornea by exposure to uncontrolled excimer laser radiation, see Taboada et al, "Response of the Corneal Epithelium to ArF Excimer Laser Pulses," 40 Health Physics 677-83 (1981)).

One technique for corneal reshaping using an excimer laser photoablation apparatus comprises controlling the reprofiling operation by varying the size of the surface area to which the pulses of laser energy are applied. In one embodiment, a beam-shaping stop or window is moved axially along the beam to increase or decrease the area of the light incident on cornea. By progressively altering the size of the exposed region, the desired photoablation profile is established on the corneal surface. For further details on this technique, see Marshall et al, "Photo-Ablative Reprofiling of the Cornea Using an Excimer Laser: Photorefractive Keratectomy," 1 Lasers in Ophthalmology, 21-48 (1986), and the commonly-owned, copending U.S. Patent Applications Ser. Nos. 869,335 and 905,156.

Another technique for corneal reshaping involves the use of a laser photoablation apparatus in which a beam-shaping mask is disposed between the laser and the surface. The mask provides a predefined profile of resistance to laser radiation erosion by selectively absorbing some of the laser radiation while permitting the remainder to be transmitted to the surface in accordance with the mask profile. For further details of such erodible masking techniques, see copending US-A-4856513 and WO-A-8705496, incorporated herein by reference.

It also has been suggested that controlled ablative photodecomposition of one or more selected regions of a cornea can be performed by scanning the cornea with a beam from an excimer laser. For example, U.S. Patent 4,665,913, issued to L'Esperance suggests correcting myopia and hyperopia by repeatedly scanning the cornea with an excimer laser beam to penetrate the corneal stroma and induce resculpting. Because of the scanning action, it is necessary for L'Esperance to bring his laser beam to a small spot, typically a rounded square dot of size 0.5 mm by 0.5 mm.

L'Esperance suggests that myopic and hyperopic conditions can be reduced by altering the curvature of the outer surface of the cornea by repeatedly scanning the cornea with an excimer laser beam having this standard, small spot size but varying the field which is scanned during successive scans, so that some areas of the cornea are scanned more often than others. In this way, it is claimed, the surface can be eroded by different amounts depending on the number of times the spot scans the surface. Additionally, he suggests that certain severe myopic and hyperopic conditions may be treated with a reduced removal of tissue by providing the outer surface of the cornea with a new shape having Fresnel-type steps between areas of the desired curvature.

In practice, complex apparatus is required to cause a pulsed laser beam to scan with the precision required if the eroded surface is to be smooth. Thus, if successive sweeps of a scan overlap, there will be excessive erosion in the overlap area, whereas if they fail to meet, a ridge will be left between the sweeps. The pulsed nature of excimer laser radiation also tends to exacerbate this problem. Additionally, the scanning method is inherently time-consuming even with highly refined techniques and apparatus, since the laser beam is only eroding a very small part of the total area to be treated at any given moment. Furthermore, such a scanning system can cause rippling effects on relatively soft materials such as corneal tissue.

It is therefore an object of the present invention to provide an apparatus and a method for eroding a surface using a laser which does not require scanning of the area of the surface to be eroded.

Another technique for corneal reshaping involves the use of a laser photoablation apparatus in which the size of the area on the surface, to which the pulses of laser energy are applied, is varied to control the reprofiling operation. In one preferred embodiment, a beam-shaping stop or window is moved axially along the beam to increase or decrease the region of cornea on which the laser radiation is incident. By progressively varying the size of the exposed region, a desired photoablation profile is established in the surface. For further details on this technique see also, Marshall et al., "Photo-ablative Reprofiling of the Cornea Using an Excimer Laser: Photorefractive Keratoctomy", Vol. 1, Lasers in Ophthalmology, pp. 21-48 (1986) herein incorporated by reference.

Although this technique for varying the size of the exposed region is a substantial improvement over physical shaping (i.e., scalpel) techniques and laser spot scanning protocols, a considerable number of optical elements and control systems still are required for precise operation, particularly on human corneal tissue. There exists a need for better and simpler procedures for shaping surfaces, particularly the surfaces of biological tissues, such as corneal tissue.

It is an object of the present invention to provide an apparatus and method whereby laser techniques can be applied to the eye to effect corrections of refractive errors while minimizing the surgical risks.

According to the present invention, there is provided a masking apparatus for use in laser reprofiling of corneal tissue comprising a support structure (40) having a surface (52) shaped for fixation upon a cornea and a light-restricting mask connected to the support structure thereby to be disposed above the cornea, characterized in that the mask comprises a variable light restriction means (42) which is operative to reprofile the corneal tissue incrementally over time by varying the size of the corneal area exposed to laser radiation, and in that the apparatus includes a control means (30) for regulating laser impingement on the corneal tissue, whereby upon irradiation of the mask a portion of the laser radiation is selectively transmitted by said light restriction means (42) to the cornea in accordance with the control means (30) to selectively erode said corneal tissue consequential on cumulative differences in exposure thereof to ablative laser radiation.

The invention includes a masking apparatus comprising a support structure adapted for fixation upon a surface to be eroded and reprofiled and a mask connected to the support structure and disposed above the said surface. The mask comprises a light restriction element, and a control means or element for regulating laser impingement on the said surface. Upon exposure of the mask to laser radiation, a portion of the laser radiation is selectably transmitted by the light restriction element to the surface, in accordance with the control means, resulting in the selective erosion of the surface.

In one embodiment, the light restriction element comprises an adjustable iris having a plurality of overlapping leaves which define a variable sized aperture. The iris is preferably adjusted using as control means a motor element, e.g. a stepper motor.

In another embodiment of the invention, the light restriction element comprises a plate having a plurality of selectable apertures of varying diameter. For example, the apertures can be generally circular or annular depending on whether a hyperopia or myopia-correcting change in the curvature of the cornea is desired.

Alternatively, the plate can have a plurality of elongate apertures of different widths, i.e., oval or rectilinear, such that the apparatus can be used to correct astigmatic refractive errors in the curvature of the cornea. In this embodiment, a motor element can be used to displace, e.g. to rotate the plate and thus present different apertures to the laser radiation.

The apparatus of the invention can also comprise a microprocessor connected to the control means or element. The microprocessor stores and executes the sequence of steps necessary to reprofile the surface, e.g. corneal tissue.

US-A-4,648,400 discloses an apparatus and a method according the preambles of claims 1 and 10 respectively with which this invention possesses similarities. This US patent discloses an ophthalmic surgery system for performing corneal keratotomies and resectomies using a masking apparatus. The masking apparatus comprises a support structure having a surface shaped for fixation on a cornea and a light-restricting mask connected thereto for disposition above the cornea. The masking apparatus comprises several elements by which passage of light from a laser to the cornea is allowed or prevented. First, the surface shaped for fixation on the cornea has an array of radial slots uniformly spaced about an optical axis. Second, there is a shutter plate mounted above said shaped surface for rotation about the optical axis, the shutter plate having slots corresponding to those in the shaped surface. The shutter plate is normally located in closed offset rotational position, i.e. so that it prevents laser light reaching the shaped surface. Superposed over the shutter is a top plate which is also rotational about the optical axis, the top plate possessing at least one slit to pass a precisely defined beam of laser light. The slit is of a fixed, precise length and width.

In use, the support structure of the US patent is releasably secured to the surface (cornea) to be operated upon. The top plate is positioned with its slit in registry with one of the slots in the shaped surface. With the laser functioning, the shutter is opened and the cornea irradiated - via the aligned slits/slots in the top plate, shutter and shaped surface - for as many pulses of radiation as are needed to form a slit-like incision of the required depth in the cornea. The shutter is then closed. After rotating the top plate and shutter relative to the fixed shaped surface, the cornea is irradiated via another set of aligned slits/slots. The process is repeated to create a series of radial incisions in the cornea.

In contrast, in the present invention a mask comprising a variable light restriction means is provided, and it is adjusted to regulate the radiant energy incident on a cornea - or other surface to be reprofiled - by varying the size of the exposure area incrementally over time.

Also according to the present invention, there is provided a method of reprofiling a surface of a non-living object comprising the steps of:
providing a masking apparatus comprising a support structure (40) having a surface (52) shaped to fit the surface to be reprofiled and a light-restricting mask connected to the support structure so as to be spaced from the surface (52) and interposed in a beam propagation path from a radiant energy source (12) to the surface (52);
releasably securing the support structure to the said surface to be reprofiled with the mask (16) spaced therefrom;
characterized by:
the mask (16) comprising a variable light restriction means (42), and by operating a control means (30) to adjust the light restriction means (42) to regulate the radiant energy incident upon the surface to be reprofiled, by varying the size of the exposure area on the surface incrementally over time;
whereby a portion of the surface is selectively ablated by cumulative differences in exposure to the incident radiant energy in accordance with the adjustment of the light restricting means (42), the latter being an iris diaphragm or an opaque plate having a plurality of selectable apertures of ranging areas.

By adjusting the light restriction element, for instance by actuating the leaves of an iris, or by replacing one aperture in an apertured plate with another, the radiant energy incident upon the surface can be adjusted, whereby a portion of the surface is selectively ablated by the incident radiant energy in accordance with the adjustment of the light restriction element.

The methods and apparatus of the present invention are particularly well suited for controlled reprofiling of the cornea, particularly a region known as Bowman's membrane, which lies immediately below the uniform, extremely thin, epithelial layer of the cornea. The epithelial layer is very rapidly ablated on exposure to the laser light and the laser source is preferably an excimer laser, such as a UV Argon Fluoride laser operating at about 193 nanometers, which does not penetrate through the cornea. A minimum laser irradiance level is essential for ablation, but it is preferred not greatly to exceed this minimum threshold.

The pulse repetition rate for the laser may be chosen to meet the needs of each particular application. Normally, the rate will be between 1 and 500 pulses/sec., preferably between 1 and 100 pulses/sec.

Suitable irradiation intensities vary depending on the wavelength of the laser and the nature of the irradiated object. For a given wavelength of laser energy applied to any given material, there will typically be a threshold value of the energy density below which significant erosion does not occur. Above the threshold density, there will be a range of energy density above which increasing energy densities give increasing depths of erosion, until a saturation level is reached. For increases in energy density above the saturation value, no significant increase in erosion occurs.

The threshold value and the saturation value will vary between wavelengths of laser energy and between target surface materials. However, for any particular laser wavelength and any particular material, the values can be found readily by experiment. For example, in ablation of the Bowman's membrane of the cornea alone or the membrane and he underlying corneal stroma by energy of wavelength 193nm (the wavelength obtained from an ArF excimer laser), the threshold value is about 50 mJ per cm² per pulse, and the saturation value is about 250 mJ per cm² per pulse. There appears to be little benefit in exceeding the saturation value by more than a small factor, and suitable energy densities at the corneal surface are 50 mJ per cm² to one J per cm² per pulse for a wavelength of 193nm.

The threshold value can vary very rapidly with wavelength. At 157nm, which is the wavelength obtained from a F₂ laser, the threshold is about 5 mJ per cm² per pulse. At this wavelength, suitable energy densities at the corneal surface are 5 mJ per cm² to one J per cm² per pulse.

Most preferably, the laser system is used to provide an energy density at the surface to be eroded of slightly less than the saturation value. Thus, when eroding the cornea with a wavelength of 193 nm (under which conditions the saturation value is 250 mJ per cm² per pulse), it is preferable to provide to the erodible mask and cornea pulses of an energy density of 100 to 200 mJ per cm² per pulse. Typically, a single pulse will erode a depth in the range 0.1 to 1 micrometer of collagen from the cornea.

The invention will next be described by way of example in connection with certain illustrated embodiments; however, it should be clear that those skilled in the art can make various modifications, additions and subtractions without departing from the scope of the claimed invention. For example, the invention can be used in connection with corneal transplants or synthetic inlays.

Moreover, the teaching of the present invention can be applied to other biological tissues requiring reprofiling, including lenticular implants, ligaments, cartilage, and bone. The apparatus and method of this invention are applicable also to non-surgical techniques of profiling non-biological surfaces e.g. for the production of synthetic inlays and lenses.

The invention will now be described by way of example with reference to the accompanying drawings:
FIG. 1 is a schematic illustration of an apparatus for practicing a method of reprofiling the surface of a cornea, in accordance with the invention;
FIG. 2 is a side view of a first embodiment of a masking apparatus in accordance with the invention;
FIG. 3 is a bottom view of the masking apparatus shown in FIG. 2;
FIG.4 is a side view of a second embodiment of a masking apparatus in accordance with the invention;
FIG. 5 is a top view of a first apertured plate useful in the masking apparatus of FIG. 4;
FIG. 6 is a top view of a second embodiment of an apertured plate useful in the masking apparatus of FIG. 4;
FIG. 7 is a top view of a third apertured plate useful in the masking apparatus of FIG. 4;
FIGS. 8A-8D are schematic illustrations of how the beam-shaping element of the present invention can operate to reduce corneal curvature by the selective ablation of tissue;
FIGS. 9A-9D are schematic illustrations of how the beam-shaping element of the present invention can operate to increase corneal curvature by the selective ablation of tissue;
FIG. 10 is an overall schematic view of a clinical system for measurement and reprofiling operations during laser corneal surgery; and
FIG. 11 is flow diagram illustrating a method of feedback control in accordance with the invention.

The invention provides a masking apparatus suitable for use in laser reprofiling of corneal tissue. The apparatus can be secured to the corneal surface thus decreasing, or eliminating, ablation errors resulting from movement of either the ocular surface or the masking apparatus itself.

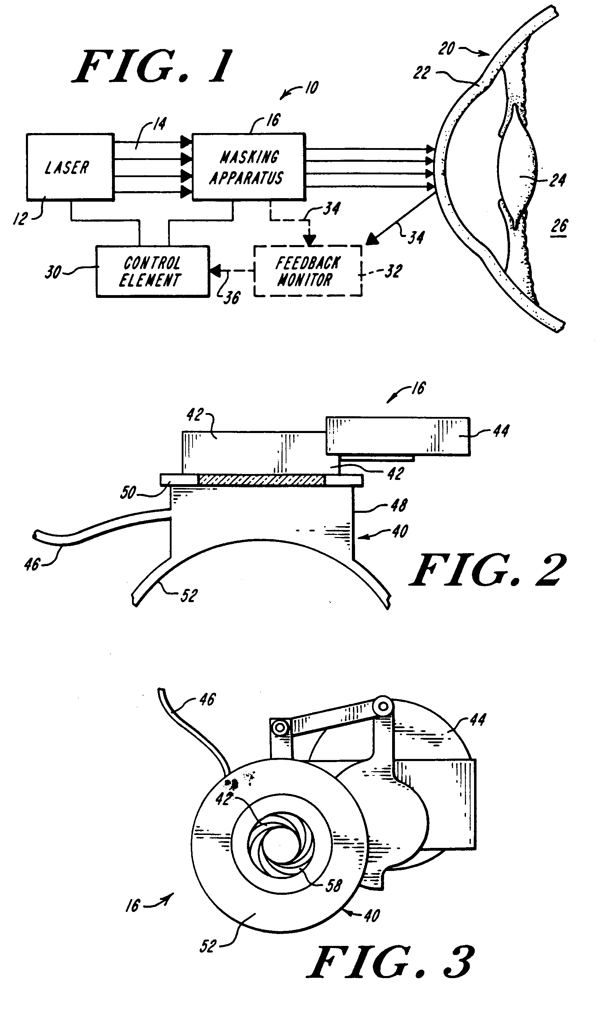

FIG. 1 depicts a laser system 10 including a laser 12 which provides a radiation output 14 to a masking apparatus 16. The masking apparatus 16 imposes a defined ablation profile onto the surface of the eye 20 to effect reprofiling. Eye 20 includes a cornea 22, lens 24 and a vitreous body 26 which together define an optical path for vision.

The laser system 10 is powered by a power supply unit (not shown) and can be controlled by a control element 30. Control element 30, i.e., a microprocessor, can function to present selected apertures to the laser radiation 14, and, in some embodiments, adjust laser 12 to produce pulses of light at specified frequencies and/or intensities. To further control the laser 12, a feedback monitor 32 can be provided which receives measurement signals 34 from the masking apparatus 16 and/or the cornea 22 during the ablation process. The feedback monitor 32 generates a feedback control signal 36 which is communicated to the control element 30 for controlling the masking apparatus 16 and, where applicable, the laser 12.

A particular advantage of the present invention lies in the ability to position the masking apparatus in close proximity to the eye. By incorporating the masking apparatus into an eye piece which is affixed to the eye, alignment problems are minimized, since the mask and the eye are secured together and the laser need only deliver ablative radiation to the masking apparatus. Thus, alignment of the laser beam becomes less critical so long as the beam is sufficiently wide to insure that the masking apparatus is irradiated throughout the reprofiling operation.

The present invention therefore permits the clinician to engage in a simple and straightforward reprofiling operation. The clinician need only align the masking apparatus/eye piece with the patient's eye. The problems of precisely aligning a laser beam with the eye are minimized, and the need for alignment with further optical elements, such as a spatially separated mask, or imaging lenses, are likewise avoided.

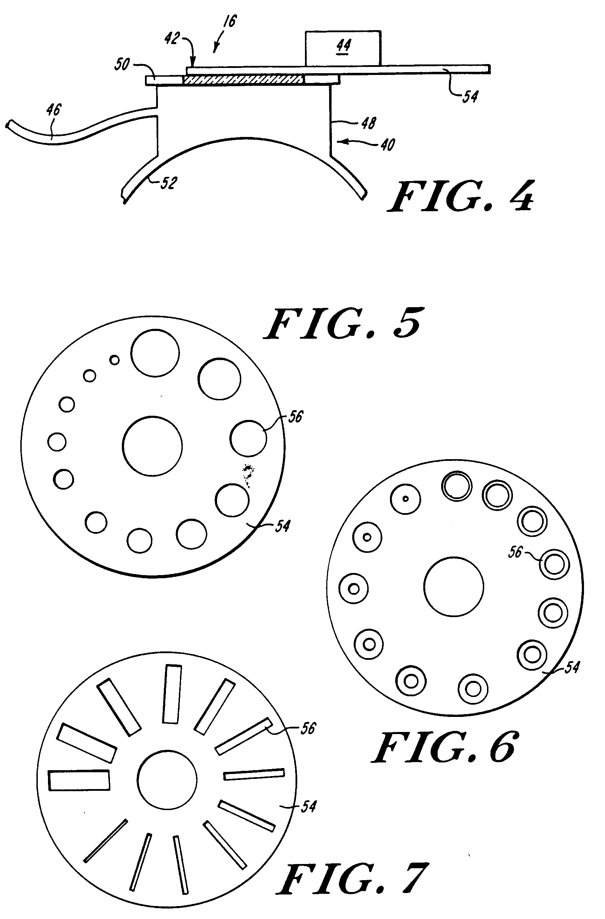

FIGS. 2, 3, and 4 depict two embodiments of the masking apparatus 16. Generally, apparatus 16 includes an eyepiece 40, a light restriction element 42, a motor 44, and a flexible tube 46. The eyepiece 40 is hollow and has rigid vertical walls 48, and a horizontal stage 50. Preferably the base 52 of eyepiece 40 is shaped to be complementary to the curvature of cornea 22. The masking apparatus 16 can be placed upon the sclera of the cornea 22 and secured using a vacuum suction drawn through flexible tube 46 leaving the corneal surface unobstructed.
The vacuum is sufficient to secure apparatus 16 to the cornea 22 without distorting the shape of cornea 22. Alternatively, the eyepiece 40 can be manually secured to the eye or held in place by pressure or by a suitable adhesive, or combinations of these means. The light restriction element 42 is affixed to the horizontal stage 50. Element 42 can, as shown in the embodiment of FIGS 2 and 3, be an iris having a plurality of overlapping leaves 58. The leaves 58, which are actuated by motor 44, pivot so as to open out in a direction away from the optical axis. Alternatively, as shown in the embodiment of FIG. 4, the light restriction element 42 can be a plate 54 having selectable apertures 56. The plate 54 illustrated in Fig. 4, and also in Figs. 5, 6 and 7, is displaced rotatively but it could be designed to be otherwise displaceable to bring selected apertures into coincidence with the optical axis. In operation motor 44 rotates plate 54 to present a selected aperture 56 to the laser radiation 14. Motor 44 can be any electrically driven motor and is preferably a stepper motor.

FIGS. 5, 6, and 7 depict embodiments of plate 54 useful in the embodiment of the invention of FIG. 4. Preferably, plate 54 is an annular ring. The apertures 56, i.e., beam-shaping stops or windows, are successively distributed around the peripheral edge of the plate 54. The particular plate 54 employed in a given operation will depend upon the size and type, i.e., hyperopic, myopic, or astigmatic, of the refractive error being treated. In addition to apertures 56, the plate 54 can have indexing insignia to identify particular apertures and alignment into position along a projection axis of the laser.

Plate 54 of FIG. 5 is used to effect myopic-correcting ablation to the cornea 22. The apertures 56 of plate 54 preferably have a substantially circular shape. Other shapes can be employed, e.g., ellipsoidal, to produce ablation of similarly shaped aberrations in the curvature of the cornea. In operation, the successive transmission of light through the apertures 56, i.e., greater to lesser diameter, creates a fresnel shaped ablation profile in the cornea 22.

FIG. 6 depicts a plate 54 useful to effect hyperopic-correcting ablation to the cornea 22. The apertures 56 of this embodiment of plate 54 are annular. Preferably, the inner radii of the apertures progressively changes while the outer radii remains substantially constant. In operation, the successive transmission of light through the apertures, i.e., lesser to greater inner radii, creates a "hill-like" ablation profile.

FIG. 7 depicts a plate 54 useful effect astigmatic-correcting ablation to the cornea 22. The apertures 56 of this embodiment of the plate 54 are oval or rectilinear. The longitudinal axes of the apertures 56 can be aligned at any angle relative to the radii of plate 54 to correct a particular astigmatic refractive error. By ablative laser pulsing with the succession of rectilinear apertures, a cylindrical cumulative ablation penetration profile can be produced.

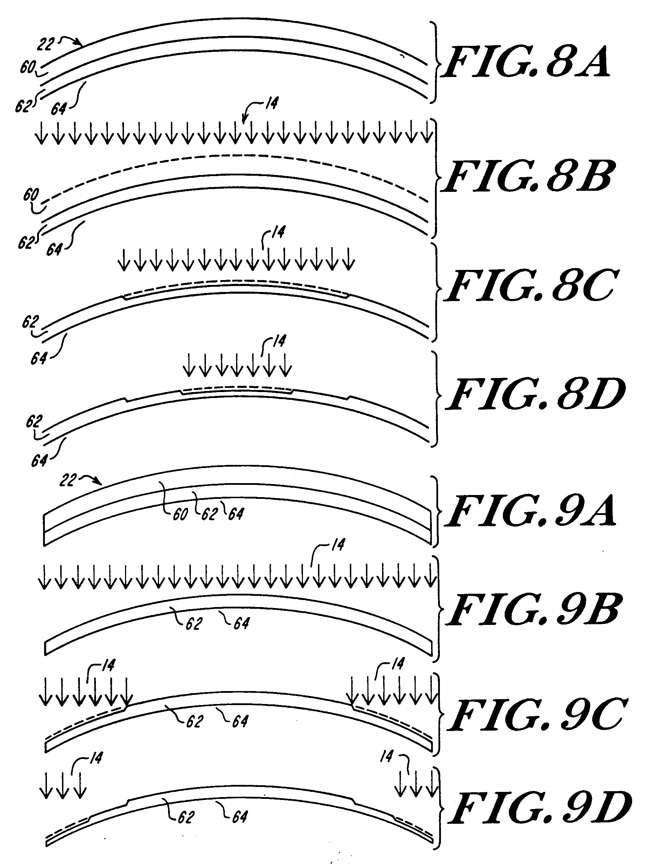

With reference to FIGS. 8A-D, the overlying epithelium 60 of the cornea 22 typically is removed prior to, or as part of, the reprofiling process and can, for example, be ablated by the initial pulses of the laser. After erosion of the epithelium 60, the laser can erode the exposed Bowman's membrane 62 to effect a permanent change in the shape of the cornea. Alternatively, the masking apparatus can be employed to impart initially a desired change in curvature in the epithelium. Upon additional irradiation, this change in the curvature of the epithelium can then be transferred to the Bowman's membrane region of the eye and/or the upper strata of the stroma. By avoiding substantial penetration into the corneal stroma 64, the present invention minimizes the disturbance of the fibrillar regularity of the substantia propria of the corneal stroma reducing the trauma and risk of the procedure. Once the operation is completed, the epithelium 60 reforms by the natural healing process.

FIGS. 8A-8D are schematic illustrations of how the beam-shaping means of FIGS. 2, 3 and 4 can operate to reduce the curvature of the cornea 22 by selective ablation of tissue from Bowman's membrane. In FIG 8A, the intact surface layers of the cornea 22 are shown comprising the epithelium 60, Bowman's membrane 62 and the initial layers of the corneal stroma 64. In FIG. 8B, a large aperture is employed to ablate, using incident laser beam 14, all or a substantial portion of the epithelial layer 60 of the cornea 22 in a region of the optical zone so as expose the surface of Bowman's membrane 62. A first ablation region of wide cross-sectional area is then created in Bowman's membrane 62 as shown in FIG. 8C. A narrower region of further ablation is then created as shown in FIG. 8D to create a flattened curvature. Of course, a substantially greater number of ablation steps is normally employed to achieve a smooth curve and minimize the step-effects. Upon completion of the laser surgery, the epithelium 60 regrows with a uniform thickness and produces a new corneal curvature determined by the new curvature of the remaining Bowman's membrane 62.

FIGS. 9A-9D are schematic illustrations of how the beam-shaping element of FIGS. 2, 3 and 4 can operate to increase the curvature of the cornea by selective ablation of tissue from Bowman's membrane. The process is essentially analogous to the procedure described above in connection with FIGS. 8A-8D except that an aperture 56 having a central beam-stopping region, e.g., similar to that described with regard to FIG. 6, is employed to create a ring-like ablation zone, preferably located on the periphery of the optical zone. The ablation creates a "hill-like" profile which increases the curvature of the cornea 22 upon regrowth of the epithelial layer over the resculpted Bowman's membrane 62. Again, as noted in connection with FIGS. 8A-D, the epithelium can either be removed initially (e.g. by laser ablation) prior to reprofiling, or a change in curvature can be implanted first to the epithelium and then transferred to the Bowman's membrane and/or stromal regions.

In both instances, the correction for myopia (illustrated in FIGS. 8A-8D) and the correction for hyperopia (illustrated in FIGS. 9A-9D) can be conducted by ablation of Bowman's membrane 62 with minimal disturbance (or penetration) into the corneal stroma 64. An improved and less traumatic procedure which avoids surgical ablation of the highly ordered fibrillar structure of the stroma 64 is thus provided.

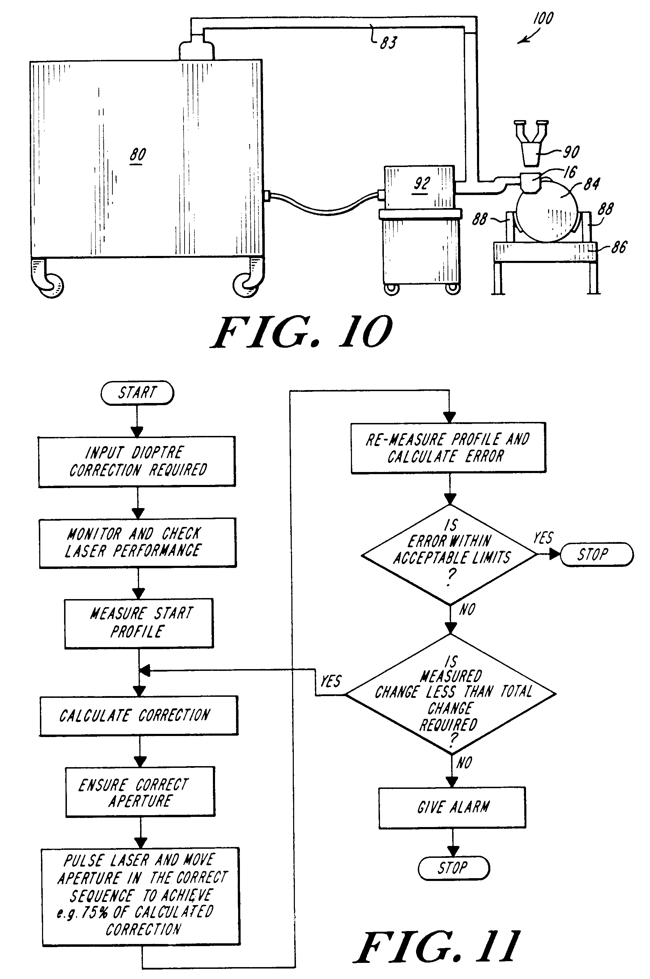

Figure 10 illustrates a clinical apparatus 100 for reprofiling the cornea of a human eye. A laser and associated control circuitry is contained in a housing 80. The beam-forming optics, for providing a beam of desired shape and size, can also be contained within the housing 80 together with the laser power supply control circuits. An optical waveguide 83, which can be flexible or rigid and includes suitable mirrors, prisms, and lenses, is provided to transmit the laser beam output from the housing 80 to the patient 84. The patient 84 is lying face-upwards on an operating table 86 with masking apparatus 16 secured to the surface of cornea 22. The operating table 86 will support the patient's head against vertical movement. If desired, side supports 88 may also be provided to restrain lateral movement of the patient's head.

Prior to the operation, the light restricting masking means as described above are preset based on measurements of the patient's eye and designed to impart the desired refractive correction upon use. Next, the above-described masking apparatus 16 is placed upon the sclera of the cornea 22 and secured using a vacuum suction drawn through flexible tube 46. Laser 12 is then activated and the surface of the cornea 22 is exposed to radiation as permitted by the beam-stopping optics.

During the operation, the eye can be observed using a surgical microscope 90 which is supported above the patient by any convenient means. The surgical microscope 90 can be connected to the masking apparatus 16, but will more normally be separated therefrom and supported by an arm (not shown) from the ceiling or by a cantilever (not shown).

A measuring device 92 can also be employed in conjunction with the apparatus 100 to measure the changes in the curvature of the cornea during and following ablation. The measuring device can take the form of a commercially-available keratometer or other suitable device, and can be connected, as shown in Figure 10, directly to the laser optical path or, preferably, be movable by the operator into the position occupied by the surgical microscope 90.

The measuring device 92 can further provide the feedback control signal 36, as shown in Figure 1, whereby information from optical or other inspection of the surface which is being exposed to laser erosion is used to control the light restriction element 42 and, in some embodiments, the duration and amplitude of the pulses supplied by the laser 12. In particular, the measuring device 92 can be tuned so as to assist in the production of the desired degree of erosion of the surface by each pulse.

FIG. 11 provides a flow diagram of the control element 30 during feedback control operation of the masking apparatus of FIGS. 2, 3, and 4. The control element 30 is preferably provided at least in part with a microprocessor or microcomputer which has been programmed with the appropriate diopter, or more complex shape, change desired. In this method, a commercially available measuring device 92, i.e., keratometer, can be used to initially confirm that the corneal profile has not been affected by the suction used to secure masking apparatus 16. Subsequently, beam dimension control, and light restriction element size is operated to impart a desired change in the profile of the corneal surface.

In operation, the control element 30 can be programmed with parameters selected to produce 75 percent of the desired final correction to the corneal surface. After a preselected number of pulses, the cornea is again measured using the measuring device 92 and the exact remaining correction determined. The laser system 10 is then adjusted and operated again, in a similar manner to that just described, with the parameters selected to produce 75 percent of the correction remaining to be made. The process is repeated until the correction remaining to be made is considered to lie within acceptable limits.

Further background information useful to a fuller understanding of the present invention can be found in the following articles by one of the present inventors and colleagues: Marshall et al. "Photablative Reprofiling of the Cornea using an Excimer Laser: Photorefractive Keratectomy," Vol. 1 Lasers in Ophthalmology, pp 21-48 (1986); Tuft et al. "Stromal Remodelling following Photorefractive Keratectomy," Vol. 1 Lasers in Ophthalmology, pp 177-183 (1987); and Munnerlyn et al. "Photorefractive keractectomy: A Technique for Laser Refractive Surgery," Vol. 14 J. Cataract Refract. Surgery, pp. 46-52 (1988), herein incorporated by reference.

The invention may be embodied in other specific forms without departing from the scope of the following claims.

US Patent Application Serial No. 905,156 corresponds to European Patent Application No. 86306916.7, publication No. 224 322.

US Patent Application Serial Nos. 019,200 now US Patent No. 4,856,513 and 124,101 correspond to European Patent Application No. 87902116.0 (PCT/GB 87 00193, published under the number WO 87/05496).

## Claims

1. A masking apparatus for use in laser reprofiling of corneal tissue comprising a support structure (40) having a surface (52) shaped for fixation upon a cornea and a light-restricting mask connected to the support structure thereby to be disposed above the cornea, characterized in that the mask comprises a variable light restriction means (42) which is operative to reprofile the corneal tissue incrementally over time by varying the size of the corneal area exposed to laser radiation, and in that the apparatus includes a control means (30) for regulating laser impingement on the corneal tissue, whereby upon irradiation of the mask a portion of the laser radiation is selectively transmitted by said light restriction means (42) to the cornea in accordance with the control means (30) to selectively erode said corneal tissue consequential on cumulative differences in exposure thereof to ablative laser radiation.

2. The apparatus according to claim 1, wherein the light restriction means (42) comprises an adjustable iris having a plurality of overlapping leaves (58) which define an aperture of variable size.

3. The apparatus according to claim 2, wherein the control means (30) includes a motor means (44) for adjusting the placement of the leaves (58) of the iris.

4. The apparatus according to claim 1, wherein the light restriction means (42) comprises a plate (54) having a plurality of selectable apertures (56) of varying areas.

5. The apparatus according to claim 4, wherein the plate (54) comprises a plurality of substantially circular apertures (56) of different areas.

6. The apparatus according to claim 4, wherein the plate (52) comprises a plurality of annular apertures (56) having inner light-blocking regions of different areas.

7. The apparatus according to claim 4, wherein the plate (52) comprises a plurality of elongate apertures (56) of different width.

8. The apparatus according to claim 4, 5, 6 or 7, wherein the control means (30) comprises a motor means (44) for displacing, e.g. for rotating the plate (52) in order to present a selected aperture to said laser radiation.

9. The apparatus according to any of the preceding claims, further comprising a microprocessor means connected to the control means (30) for storing sequences of steps to reprofile corneal tissue and for instructing the control means in the execution of such steps.

10. A method of reprofiling a surface of a non-living object comprising the steps of:
providing a masking apparatus comprising a support structure (40) having a surface (52) shaped to fit the surface to be reprofiled and a light-restricting mask connected to the support structure so as to be spaced from the surface (52) and interposed in a beam propagation path from a radiant energy source (12) to the surface (52);
releasably securing the support structure to the said surface to be reprofiled with the mask (16) spaced therefrom;
characterized by:
the mask (16) comprising a variable light restriction means (42), and by operating a control means (30) to adjust the light restriction means (42) to regulate the radiant energy incident upon the surface to be reprofiled, by varying the size of the exposure area on the surface incrementally over time;
whereby a portion of the surface is selectively ablated by cumulative differences in exposure to the incident radiant energy in accordance with the adjustment of the light restricting means (42), the latter being an iris diaphragm or an opaque plate having a plurality of selectable apertures of ranging areas.

## Patentansprüche

1. Eine Abdeckvorrichtung zur Verwendung bei der Laser-Korneagewebekorrektur, die einen Träger (40) mit einer Oberfläche (52), die für die Fixierung auf einer Kornea geformt ist, und eine Lichtbegrenzungsvorrichtung aufweist, die mit dem Träger verbunden ist und auf diese Weise über der Kornea angeordnet wird, dadurch gekennzeichnet, daß die Abdeckvorrichtung eine regelbare Lichtbegrenzungsvorrichtung (42) umfaßt, die bei Korrektur des Korneagewebes inkremental mit der Zeitdauer wirksam wird, indem die Größe der dem Laserstrahl exponierten Korneafläche variiert wird, und dadurch, daß die Vorrichtung eine Steuerungseinheit (30) zur Regulierung des Laser-Einfalls auf das Korneagewebe umfaßt, wodurch bei Bestrahlung der Abdeckvorrichtung selektiv ein Teil der Laserstrahlung von besagter Lichtbegrenzungsvorrichtung (42) in Übereinstimmung mit der Steuerungseinheit (30) zur Kornea geleitet wird zum selektiven Abtragen besagten Korneagewebes entsprechend den kumulativen Differenzen in der Exposition der Kornea mit dem abtragenden Laserstrahl.

2. Eine Vorrichtung gemäß Anspruch 1, worin die Lichtbegrenzungsvorrichtung (42) eine regulierbare Irisblende mit zahlreichen überlappenden Blättern (58) aufweist, die eine Öffnung mit regelbarer Größe definieren.

3. Eine Vorrichtung gemäß Anspruch 2, worin die Steuerungseinheit (30) ein Antriebsteil (44) zum Regulieren der Stellung der Irisblätter (58) umfaßt.

4. Eine Vorrichtung gemäß Anspruch 1, worin die Lichtbegrenzungsvorrichtung (42) eine Scheibe (54) mit zahlreichen wählbaren Öffnungen (56) unterschiedlicher Fläche aufweist.

5. Eine Vorrichtung gemäß Anspruch 4, worin die Scheibe (54) zahlreiche im wesentlichen kreisförmige Öffnungen (56) unterschiedlicher Fläche aufweist.

6. Eine Vorrichtung gemäß Anspruch 4, worin die Scheibe (52) zahlreiche ringförmige Öffnungen (56) mit innenliegenden, das Licht blockierenden Bereichen unterschiedlicher Fläche aufweist.

7. Eine Vorrichtung gemäß Anspruch 4, worin die Scheibe (52) zahlreiche Schlitzöffnungen (56) unterschiedlicher Weite aufweist.

8. Eine Vorrichtung gemäß Anspruch 4, 5,6 oder 7, worin die Steuerungseinheit (30) ein Antriebsteil (44) zum Verstellen, z. B. zum Drehen der Scheibe (52), um eine ausgewählte Öffnung in besagten Laserstrahl zu schieben, aufweist.

9. Eine Vorrichtung gemäß einem der vorausgehenden Ansprüche, die des weiteren einen an die Steuerungseinheit (30) angeschlossenen Mikroprozessor zur Speicherung der Schrittfolge für die Korneagewebekorrektur und als Befehlsgeber der Steuerungseinheit bei Ausführung derartiger Schritte umfaßt.

10. Ein Verfahren zur Korrektur einer Oberfläche eines nicht-lebenden Objekts, das die Schritte umfaßt:
Bereitstellen einer Abdeckvorrichtung, die einen Träger (40) mit einer Oberfläche (52), die passend mit der zu korrigierenden Oberfläche geformt ist, und eine Lichtbegrenzungsvorrichtung umfaßt, die mit dem Träger verbunden ist, damit ein Abstand zur Oberfläche (52) eingehalten wird, und die in einen Strahlengang zwischen der Strahlenquelle (12) und der Oberfläche (52) gebracht wird;
lösbares Befestigen des Trägers an besagter, zu korrigierender Oberfläche mit der Abdeckung (16), die davon beabstandet ist;
gekennzeichnet durch;
die Abdeckung (16), eine regelbare Lichtbegrenzungsvorrichtung (42) aufweist, und durch das Bedienen einer Steuerungseinheit (30) zum Einstellen der Lichtbegrenzungsvorrichtung (42) zum Regulieren der Energie der einfallenden Strahlung auf die zu korrigierende Oberfläche, durch Variieren der Größe der Expositionsfläche auf der Oberfläche inkremental mit der Zeitdauer;
wodurch ein Teil der Oberfläche durch kumulative Differenzen in der Exposition mit der Energie der einfallenden Strahlung entsprechend der Einstellung der Lichtbegrenzungsvorrichtung (42) selektiv abgetragen wird, wobei das letztere ein Irisdiaphragma oder eine lichtundurchlässige Scheibe ist, die zahlreiche einstellbare Öffnungen variierender Größe aufweist.

## Revendications

1. Appareil de masquage pour l'utilisation dans le refaçonnage au laser du tissu de cornée comprenant une structure de support (40) ayant une surface (52) formée pour la fixation sur une cornée et un masque restreignant la lumière relié à la structure de support de manière à être disposé au-dessus de la cornée, caractérisé en ce que le masque comprend des moyens (42) variables de restriction de la lumière qui fonctionne pour refaçonner le tissu de cornée de manière incrémentale par rapport au temps en variant la taille de la surface de cornée exposée au rayonnement laser et en ce que l'appareil inclut des moyens de commande (30) pour réguler l'incidence du laser sur le tissu de cornée de sorte que sur une irradiation du masque une partie du rayonnement laser est transmise de manière sélective par lesdits moyens (42) de restriction de lumière vers la cornée en fonction des moyens de commande (30) pour éroder de manière sélective ledit tissu de cornée ce qui est la conséquence des différences cumulatives dans l'exposition de celle-ci au rayonnement laser ablatif.

2. Appareil selon la revendication 1, dans lequel les moyens (42) de restriction de lumière comprennent un iris ajustable ayant une pluralité de lames (58) chevauchantes qui définissent une ouverture de taille variable.

3. Appareil selon la revendication 2, dans lequel les moyens de commande (30) incluent des moyens de motorisation (44) pour ajuster le déplacement des lames (58) de l'iris.

4. Appareil selon la revendication 1, dans lequel les moyens (42) de restriction de lumière comprennent une plaque (54) ayant une pluralité d'ouvertures (56) sélectionnables de surfaces variables.

5. Appareil selon la revendication 4, dans lequel la plaque (54) comprend une pluralité d'ouvertures (56) substantiellement circulaires de différentes surfaces.

6. Appareil selon la revendication 4, dans lequel la plaque (52) comprend une pluralité d'ouvertures annulaires (56) ayant des régions internes bloquant la lumière de différentes surfaces.

7. Appareil selon la revendication 4, dans lequel la plaque (52) comprend une pluralité d'ouvertures (56) allongées de différentes largeurs.

8. Appareil selon la revendication 4, 5, 6 ou 7, dans lequel les moyens de commande (30) comprennent des moyens de motorisation (44) pour déplacer par exemple pour faire tourner la plaque (52) afin de présenter une ouverture sélectionnée audit rayonnement laser.

9. Appareil selon l'une quelconque des revendications précédentes, comprenant en outre des moyens à microprocesseur connectés aux moyens de commande (30) pour stocker des séquences d'étapes de refaçonnage de tissu de cornée et pour instruire les moyens de commande dans l'exécution de telles étapes.

10. Procédé de refaçonnage d'une surface d'un objet non vivant comprenant les étapes de :
- fournir un appareil de masquage comprenant une structure de support (40) ayant une surface (52) formée pour s'ajuster à la surface à refaçonner et un masque restreignant la lumière relié à la structure de support afin d'être espacé de la surface (52) et interposé dans un passage de propagation d'un faisceau depuis une source (12) d'énergie rayonnante vers la surface (52) ;
- fixer de manière libérable la structure de support à ladite surface à refaçonner avec le masque (16) espacé de celle-ci ;
caractérisé par :
le masque (16) comprenant des moyens (42) variables de restriction de lumière, et en commandant les moyens de commande (30) pour ajuster les moyens (42) de restriction de lumière pour réguler l'énergie rayonnante incidente sur la surface à refaçonner, en faisant varier la dimension de la surface exposée sur la surface de manière incrémentale en fonction du temps ;
de manière qu'une portion de la surface est sélectivement supprimée par des différences cumulatives dans l'exposition à l'énergie rayonnante incidente en fonction de l'ajustement des moyens (42) de restriction de lumière, ces derniers étant un diaphragme à iris ou une plaque opaque ayant une pluralité d'ouvertures sélectionnables de surface de jaugeage.
